# EUROPEAN PATENT APPLICATION

(11) **EP 4 850 725 A1**
(43) Date of publication of application: **09.12.2026**
(21) Application number: 25382576.4
(22) Date of filing: 05.06.2025
(51) Int. Cl.: A61M 11/02, A61M 35/00, B05B 7/00

(54) **LOW-PRESSURE NEBULIZERS AND METHODS TO NEBULIZE LIQUID FORMULATIONS**

(71) Applicant: Universidad del País Vasco/Euskal Herriko Unibertsitatea, 48940 Leoia, Bikaia (ES)
(72) Inventor: FERNÁNDEZ GÁMIZ, Unai, 01006 Vitoria-Gasteiz (ES); PEDRAZ MUÑOZ, Jose Luis, 01006 Vitoria-Gasteiz (ES); MORENO LOPE, Eneko Gartxot, 01006 Vitoria-Gasteiz (ES); ENRÍQUEZ RODRÍGUEZ, Lucía, 01006 Vitoria-Gasteiz (ES); SCAINI, Denis, 01006 Vitoria-Gasteiz (ES)
(74) Representative: ZBM Patents - Zea, Barlocci & Markvardsen

(57) **Abstract**

A low-pressure nebulizer is provided. The low-pressure nebulizer has a tube. The tube comprises a proximal inlet end, a distal outlet end, a gas lumen, and a liquid lumen. The proximal inlet end is configured for a pressurized gas and a liquid to enter the tube. The distal outlet end is configured for the pressurized gas and the liquid to exit the tube. The gas lumen is configured to convey the pressurized gas from the proximal inlet end to the distal outlet end. The liquid lumen is configured to convey the liquid from the proximal inlet end to the distal outlet end. The low-pressure nebulizer is configured such that the pressurized gas and the liquid are mixed outside the tube at the distal outlet end to form an aerosol. The liquid lumen comprises a main proximal segment with a first length and a first cross section, a contracted intermediate segment with a second length and a second cross section, and an expanded distal segment with a third length and a third cross section. The second cross section is smaller than the first cross section, and the third cross section is greater than the second cross section.

## Description

The present disclosure relates to a low-pressure nebulizer for liquid formulations and a method to nebulize liquid formulations.

### BACKGROUND

A liquid formulation is a mixture comprising an active agent and liquid, where the active agent is dissolved or suspended in the liquid. The mixture sometimes may further comprise stabilizers, preservatives, carriers or others, to enhance stability and effectiveness of the active agent. These liquid formulations are widely used across various fields, including pharmaceuticals (e.g., small-molecule drugs, lipid-based nanoparticles for drug delivery), biologics (e.g., proteins, monoclonal antibodies, nucleic acids, enzymes, cells), cosmetics (e.g., bioactive ingredients for skin treatments, anti-aging serums, ophthalmic care products), and agriculture (e.g., plant growth hormones, biofertilizers, pesticides).

Liquid formulations are commonly used to deliver active agents either to targeted areas or for systemic therapeutic effects. During this delivery process, nebulizers or atomizers are often employed to convert the liquid into a fine mist or aerosol, facilitating deposition at the targeted areas or enabling systemic absorption. This approach can enhance the absorption of active agents and/or improve overall therapeutic outcomes.

As an important example of liquid formulation, liquid medication is used to deliver active agents directly to specific organs or tissues of a patient's body that require treatment. Depending on the type of medication, different administration routes may be employed. For instance, when delivered through the pulmonary route, the aerosol may either act locally in lungs or be absorbed into the bloodstream to produce systemic effects, offering therapeutic benefits not only for respiratory conditions such as asthma, chronic obstructive pulmonary disease (COPD), and lung infections, but also for some systemic diseases. When administered via the nasal route, the aerosol may target the nasal passages and upper airways, or reach the brain through the olfactory bulb, bypassing the blood-brain barrier. This makes it effective for treating conditions like rhinitis or sinus infections, and for extra-nasal disorders including neurological disorders, hormonal treatments, pain management, and vaccination. Additionally, when delivered via the ophthalmic route, the medication aerosol is directly applied to the eyes, effectively treating conditions such as dry eye or ocular affections. Depending on medication types and administration route, different droplet sizes are required for the fine mist or aerosol generated by nebulizers. For example, larger droplets may be used for nasal or oral delivery, as they tend to deposit in the upper airway. In contrast, smaller droplets are better suited for deep lung deposition via pulmonary routes, allowing medication to reach the bronchioles and alveoli. Different regions of the lung may require different droplet sizes to optimize drug deposition and therapeutic effect.

Various types of nebulizers are designed to deliver liquid formulations, each utilizing distinct mechanisms for aerosol generation. Nebulization converts liquid into fine aerosol droplets using mechanisms such as mechanic pressurizers, pressurized gas, ultrasonic waves, or vibrating mesh technology. For example, a twin-fluid nebulizer may comprise a liquid medication chamber, a nozzle for directing compressed air, and a mixing chamber where the air and liquid are combined before being aerosolized. The air is forced through the nozzle at high velocity, breaking the liquid into fine droplets in the mixing chamber.

However, known nebulizers have several limitations. For example, they are often designed for general purposes and cannot efficiently modify the droplet size to accommodate different treatment requirements. Most nebulizers generate a fixed droplet size, meaning separate devices may be required for different formulations or administration routes, limiting their versatility. Additionally, many liquid formulations contain delicate active agents, such as nucleic acids, peptides, and non-viral vectors, which may be sensitive to heat and mechanical stress. Nebulizers that rely on, for example, high pressure or high-frequency vibration, can subject these sensitive compounds to damaging forces, compromising their effectiveness. Furthermore, poorly controlled aerosol plumes may result in inconsistent particle sizes, leading to inefficient active agent deposition and suboptimal treatment outcomes. These limitations are especially relevant for medical applications, where the precise and effective delivery of active agents is crucial for optimal treatment outcomes.

The present disclosure provides examples of nebulizers and methods that at least partially resolve some of the aforementioned disadvantages.

### SUMMARY

In a first aspect, a low-pressure nebulizer is provided. The low-pressure nebulizer has a tube. The tube comprises a proximal inlet end, a distal outlet end, a gas lumen, and a liquid lumen. The proximal inlet end is configured for a pressurized gas and a liquid to enter the tube. The distal outlet end is configured for the pressurized gas and the liquid to exit the tube. The gas lumen is configured to convey the pressurized gas from the proximal inlet end to the distal outlet end. The liquid lumen is configured to convey the liquid from the proximal inlet end to the distal outlet end. The low-pressure nebulizer is configured such that the pressurized gas and the liquid are mixed outside the tube at the distal outlet end to form an aerosol. The liquid lumen comprises a main proximal segment with a first length and a first cross section, a contracted intermediate segment with a second length and a second cross section, and an expanded distal segment with a third length and a third cross section. The second cross section is smaller than the first cross section, and the third cross section is greater than the second cross section.

In the disclosed nebulizer, the liquid and the pressurized gas are supplied through the liquid lumen and the gas lumen, respectively. The liquid and the pressurized gas remain separate and do not interact with each other until exiting the tube. Upon exiting the tube at the distal outlet end, the liquid is nebulized by the pressurized gas.

The liquid lumen has variable cross sections along its longitudinal direction, e.g. the first cross section in the main proximal segment, the second cross section in the contracted intermediate segment and the third cross section in the expanded distal segment. According to the continuity equation, i.e. Equation 1, for one-dimensional steady flow in fluid mechanics, an increase in cross-sectional area A results in a decrease in flow velocity v, and vice versa. This equation allows for manipulation of the liquid velocity along the liquid lumen prior to its exit and subsequent mixing with the pressurized gas. ${A}_{1} {v}_{1} = {A}_{2} {v}_{2}$ Equation 1 wherein, *A*₁ and *A*₂ respectively denote areas of a first and a second cross section, while *v*₁ and *v*₂ respectively refer to flow velocities at the first and second cross section.

Moreover, a kinetic energy *E* of a fluid flow is expressed as a function of a flow velocity *v,* as shown in Equation 2. When the liquid lumen is designed to first contract and then expand, the liquid experiences a kinetic energy loss, due to velocity reduction, after passing from the contracted intermediate segment to the expanded distal segment. This special design, as disclosed herein, enables reduction of both the liquid velocity and the kinetic energy carried by the liquid. It is noted that the reduction in kinetic energy is inversely proportional to the square of the cross-sectional expansion, whereas the reduction in flow velocity is inversely proportional to the expansion itself. In other words, kinetic energy is more sensitive to changes in the cross-sectional area of the liquid lumen than flow velocity. $E = ρ {v}^{2} / 2$ Equation 2 wherein ρ is a density of a fluid flow and v is a flow velocity.

In the disclosed nebulizer, both the kinetic energy of the liquid and the pressure of the pressurized gas play critical roles in the nebulization process. When the liquid to be nebulized possesses high kinetic energy, a correspondingly high gas pressure is required to generate sufficient shear force to break the intermolecular bonds within the liquid, thereby achieving effective nebulization. However, elevated gas pressure may also lead to increased shear stress and potential heat generation, which may compromise the integrity of delicate molecules of active agents in the liquid formulation.

By allowing the liquid to reduce its kinetic energy before mixing with the pressurized gas, a lower gas pressure may be sufficient to nebulize the liquid in the disclosed nebulizer. This reduction in gas pressure may reduce thermal and mechanical stress in the liquid caused by the high-energy impact of the pressurized gas. Consequently, the risk of structural degradation of liquid formulation is reduced. This is especially beneficial when handling delicate active agents such as nucleic acids, peptides, or non-viral vectors, as preserving their structural integrity is essential for maintaining therapeutic efficacy.

Additionally, the velocity of the liquid and the pressure of the pressurized gas at the distal outlet are crucial in shaping the aerosol plume pattern. By adjusting the geometry and configuration of the liquid lumen at the distal outlet, the liquid flow velocity can be controlled. The control on the velocity of the liquid flow in turn affects the pressure of the pressurized gas and ultimately allows modulation of the aerosol plume pattern. The plume pattern directly impacts the effectiveness of aerosol administration. For example, a widespread plume may result in a less concentrated distribution of active agents, potentially reducing treatment efficacy. With the disclosed nebulizer, the ability to fine-tune the plume pattern may enable more precise control over aerosol dispersion, enhancing the efficiency of active agent delivery and improving overall treatment outcomes.

The pressure level of the pressurized gas is a key factor in determining aerosol droplet size. Since the gas pressure required for nebulization is affected by the velocity of the liquid flow, the droplet size can also be indirectly controlled by adjusting the liquid flow velocity through modifications to the liquid lumen design. Different liquid formulations often require specific droplet sizes for optimal administration through various routes. Typically, a single nebulizer may be limited to certain formulations and administration routes due to its lack of flexibility. However, with the provided nebulizer, simply by changing the tube, it may easily adapt to accommodate a wide range of liquid formulations and administration routes, offering enhanced versatility and improved performance across diverse applications.

Furthermore, the nebulizer independently regulates the liquid and the pressurized gas, mixing them externally for nebulization. The liquid is nebulized upon contact with the pressurized gas outside the tube, rather than within the tube. This design reduces the risk of clogging and improves nebulization efficiency, making it well-suited for applications requiring fine mist generation.

A known nebulizer tube usually has a uniform shape and lumen profiles along its entire length. Compared to such known tubes, the tube design in this disclosure modifies the longitudinal profile of the liquid lumen only near the distal end of the tube, while maintaining the rest of the tube unchanged, for example, the gas lumen, the main proximal segment of the liquid lumen. This minimal modification allows the tube to effectively control the liquid velocity. In other words, the tube may not require a specialized manufacturing process, as the structural modification is both local and simple. The same materials, manufacturing techniques, and devices used for producing known tubes may be applied to the disclosed nebulizer, making the process efficient and cost-effective. Moreover, the overall design of the nebulizer is relatively simple, without requiring complex mechanical or electronic components. As a result, it may be easier to manufacture and maintain, which could lead to a lower cost compared to many existing nebulizers.

In view of the above, the low-pressure nebulizer according to the first aspect may effectively nebulize liquid formulations using low-pressure gas, while offering adjustable aerosol droplet sizes and customizable plume patterns. Additionally, it may help protect delicate active agents from structural damage, ensuring the integrity and effectiveness of the liquid formulations.

In some examples, the low-pressure nebulizer further comprises a liquid formulation, and the liquid formulation comprises an active agent. The active compound may be selected from the group consisting of: a nasal therapeutic agent, a pulmonary therapeutic agent, an ophthalmic therapeutic agent, a dermatological active ingredient, a biologic molecule, a gene therapy agent, a cell or tissue growth factor, an inhalable vaccine, a heat-sensitive biological agent, a sensitive flavoring agent, an essential oil, and a food-grade probiotic.

In a second aspect, a method to nebulize liquid formulations is provided. The method comprises:
- conveying a pressurized gas in a gas lumen from a proximal inlet end to a distal outlet end of a tube,
- conveying a liquid in a liquid lumen from the proximal inlet end to the distal outlet end of the tube, wherein the liquid flows through a main proximal segment, a contracted intermediate segment and an expanded distal segment of the liquid lumen, such that a velocity of the liquid is increased in the contracted intermediate segment relative to the main proximal segment, and the velocity of the liquid is decreased in the expanded distal segment relative to the contracted intermediate segment,
- expelling, at the distal outlet end, the pressurized gas and the liquid from the tube, such that the expelled pressurized gas and the expelled liquid mix outside of the tube to form an aerosol at the distal outlet end.

Advantages derived from this aspect may be similar to those mentioned regarding the first aspect.

In some examples, the pressurized gas operates at a pressure below 1.0 bar, for example, between 0.5 and 0.95 bar. By contrast, known jet nebulizers usually require gas pressures in the range of 1.0 to 3.0 bar. Thus, the disclosed method for nebulizing liquid formulations operates at lower gas pressures than those methods used in known jet nebulizers.

Throughout the present disclosure, the terms nebulizer and atomizer can be used interchangeably.

Throughout the present disclosure, the terms aerosol and mist can be used interchangeably.

Throughout the present disclosure, a cross section of a lumen refers to the intersection of the lumen with a plane that is perpendicular to a longitudinal axis of the lumen.

Throughout the present disclosure, a distal direction refers to a direction extending from a proximal end toward a distal end. The proximal end is the end closer to the source of the pressurized gas or liquid, and the distal end is the end closer to the nebulizing target, when the disclosed nebulizer is in use.

### BRIEF DESCRIPTION OF THE DRAWINGS

Non-limiting examples of the present disclosure will be described in the following, with reference to the appended drawings, in which:
Figure 1 schematically illustrates an inner structure of a low-pressure nebulizer according to one example of the present disclosure;
Figure 2a schematically illustrates a perspective view of a cross section of the low-pressure nebulizer of Figure 1 according to one example of the present disclosure;
Figure 2b schematically illustrates a perspective view of a cross section of the low-pressure nebulizer of Figure 1 according to a further example of the present disclosure;
Figure 3a schematically illustrates a longitudinal profile of a liquid lumen of the low-pressure nebulizer of Figure 1 according to one example of the present disclosure;
Figure 3b schematically illustrates a longitudinal profile of a liquid lumen of the low-pressure nebulizer of Figure 1 according to a further example of the present disclosure;
Figure 3c schematically illustrates a longitudinal profile of a liquid lumen of the low-pressure nebulizer of Figure 1 according to a yet further of the present disclosure;
Figure 4 schematically illustrates a low-pressure nebulizer according to a further example of the present disclosure;
Figure 5 is a block diagram of a method to nebulize liquid formulations according to one example of the present disclosure.

### DETAILED DESCRIPTION OF EXAMPLES

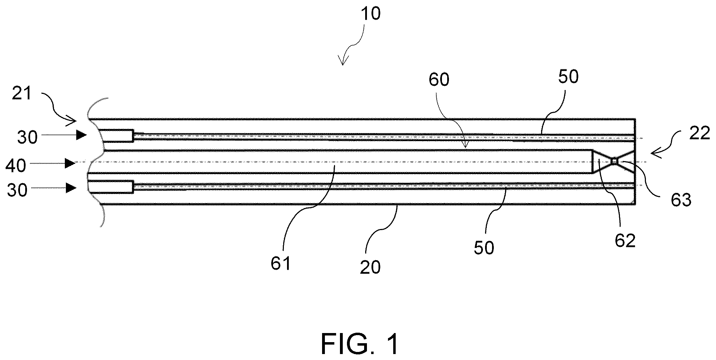

Figure 1 schematically illustrates an inner structure of a low-pressure nebulizer 10 according to one example of the present disclosure. The low-pressure nebulizer 10 has a tube 20. The tube 20 comprises a proximal inlet end 21, a distal outlet end 22, a gas lumen 50, and a liquid lumen 60. The proximal inlet end 21 is configured for a pressurized gas 30 and a liquid 40 to enter the tube 20. The distal outlet end 22 is configured for the pressurized gas 30 and the liquid 40 to exit the tube 20. The gas lumen 50 is configured to convey the pressurized gas 30 from the proximal inlet end 21 to the distal outlet end 22. The liquid lumen 60 is configured to convey the liquid 40 from the proximal inlet end 21 to the distal outlet end 22. The low-pressure nebulizer 10 is configured such that the pressurized gas 30 and the liquid 40 are mixed outside the tube 20 at the distal outlet end 22 to form an aerosol. The liquid lumen 60 comprises a main proximal segment 61 with a first length and a first cross section, a contracted intermediate segment 62 with a second length and a second cross section, and an expanded distal segment 63 with a third length and a third cross section. The second cross section is smaller than the first cross section, and the third cross section is greater than the second cross section.

In Figure 1, the gas lumen 50 and the liquid lumen 60 are parallel to a longitudinal central axis of the tube 20. While this configuration is applicable in most scenarios, there are instances where the gas lumen 50 or the liquid lumen 60 may deviate at least partially from the central axis of the tube 20. This can occur in, for example, curved or angled tube designs for targeted delivery, eccentric lumen arrangements due to space constraints, or cases where turbulent flow is enhanced by intentionally offsetting the lumens to improve nebulization efficiency.

Moreover, the example of Figure 1 shows that the gas lumen 50 and the liquid lumen 60 are arranged side by side, facilitating independent flow control in each lumen. In some cases, a coaxial configuration may be used, with the liquid lumen 60 positioned inside the gas lumen 50, providing a more compact design.

The example in Figure 1 shows that the tube 20 comprises two or more gas lumens 50, which are arranged around the liquid lumen 60. The number of gas lumens depends on the tube design and intended application. In some examples, a single gas lumen may be preferred to simplify the design and save space, while in other cases, multiple gas lumens are necessary to improve nebulization uniformity, enable adjustable gas flow by activating or deactivating specific lumens, and reduce clogging by distributing the gas flow more evenly.

Further as shown in Figure 1, the liquid lumen 50 has its central axis coinciding with the central axis of the tube 20, while the two or more gas lumens 50 may be symmetrically distributed around the central axis. In this configuration, gas flows from the gas lumens 50 may be evenly provided around a liquid flow around the liquid lumen 60 at the distal outlet end 22 to reach a more uniform mixture. In other examples, an eccentric configuration may be used, where the central axis of the liquid lumen 50 is offset from the central axis of the tube 20. This eccentric configuration may generate directional aerosol sprays, making it effective for targeted drug delivery, for example, in bronchoscopy and specialized industrial applications.

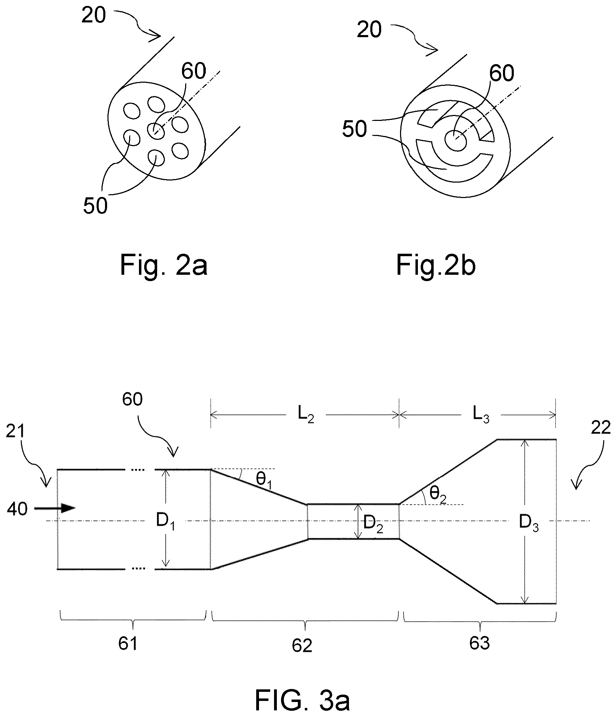

There are various possible configurations for the cross sections of the tube 20, the gas lumen 50, and the liquid lumen 60. Figure 2a provides one example, where both the tube 20 and its internal lumens, i.e. the gas lumen 50 and the liquid lumen 60, have circular cross sections. The tube 20 may comprise multiple gas lumens 50, such as the six shown in Figure 2a. In this configuration, the liquid lumen 60 is centrally positioned along the longitudinal axis of the tube 20, while the gas lumens 50 are symmetrically arranged in a circular pattern around it.

Figure 2b offers an alternative cross-sectional design. In this example, the tube 20 has two gas lumens 50, and each gas lumen has a C-shaped cross section. These gas lumens 50 are symmetrically positioned around the liquid lumen 60, maintaining a balanced and efficient flow distribution within the tube.

As mentioned above, the longitudinal profile of the liquid lumen 50 comprises three segments: the main proximal segment 61, the contracted intermediate segment 62, and the expanded distal segment 63. In the example of Figure 1, the first cross section of the main proximal segment 61 may remain constant along the first length. However, in some examples, it is possible to have variations along the first length. For example, the main proximal segment 61 may comprise contracted or expanded segments (not shown) within the first length, depending on the tube design and application. More details on the contracted intermediate segment 62 and the expanded distal segment 63 can be found in Figures 3a-3c.

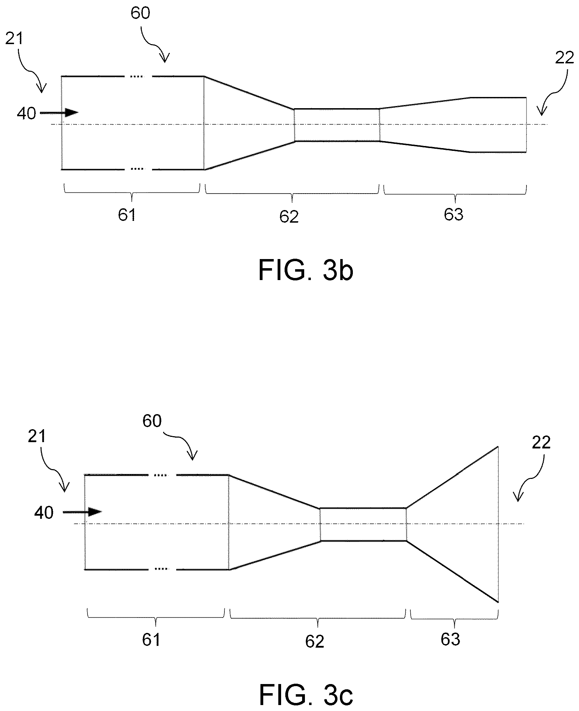

As shown in Figure 3a, the first cross section of the main proximal segment 61 has a diameter of *D*₁, the second cross section of the contracted intermediate segment 62 has a diameter of *D*₂, and the third cross section of the expanded distal segment 63 has a diameter of *D*₃. At the same time, the second length of the contracted intermediate segment 62 is labelled as *L*₂, while the third length of the expanded distal segment 63 is labelled as *L*₃. In the example of Figure 3a, *D*₂ is smaller than both *D*₁ and *D*₃, while *D*₃ is greater than *D*₁. This configuration leads to an increased liquid velocity in the segment 62 than the segments 61 and 63, and a decreased liquid velocity in the segment 63 than the segments 61 and 62. In this example, the reduced liquid velocity at the distal outlet end 22 may result in a narrower and more focused aerosol plume, as the pressurized gas becomes the dominant force in shaping the aerosol. The reduced liquid velocity may also lead to finer aerosol droplets, while potentially reducing the overall plume momentum and dispersion distance. In contrast, *D*₃ may be smaller than *D*₁ while *D*₂ is maintained as the minimum among *D*₁, *D*₂ and *D*₃, such as in the example shown in Figure 3b. In this case, the liquid velocity at the distal outlet end 22 may not necessarily be reduced compared with in the segment 61, which may result in a wider, more divergent aerosol plume with larger droplet sizes compared with the example of Figure 3a.

In both examples shown in Figures 3a and 3b, the segment 62 comprises a gradually contracting sub-segment followed by a constant sub-segment, while the segment 63 comprises a gradually expanding sub-segment followed by a constant sub-segment. The gradually contracting sub-segment decreases in cross-sectional area in distal direction, whereas the gradually expanding sub-segment increases in cross-sectional area in distal direction. The constant sub-segments maintain a uniform cross-section throughout their respective lengths. In some examples, such as the one shown in Figure 3c, the segment 63 may consist solely of an expanding sub-segment and may not include a constant sub-segment.

As shown in Figure 3a, the cross section of the contracted intermediate segment 62 may decrease in distal direction within a portion of the second length, with a constant decreasing slope θ₁. Similarly, the cross section of the expanded distal segment 63 increases within a portion of the third length with a constant increasing slope θ₂. In some examples, both θ₁ and θ₂ are within a range of 5° to 50°. A smaller slope results in a smoother transition in cross-sectional area, while a larger slope leads to a more abrupt change.

In some examples, the second length *L*₂ of the contracted intermediate segment 62 is less than 10% of an entire length of the liquid lumen 60, and the third length *L*₃ of the expanded distal segment is less than 15% of the entire length of the liquid lumen 60. The constant sub-segment of segment 62 is usually very short, which, in some cases, may account for less than 2% of the total length of the liquid lumen 60.

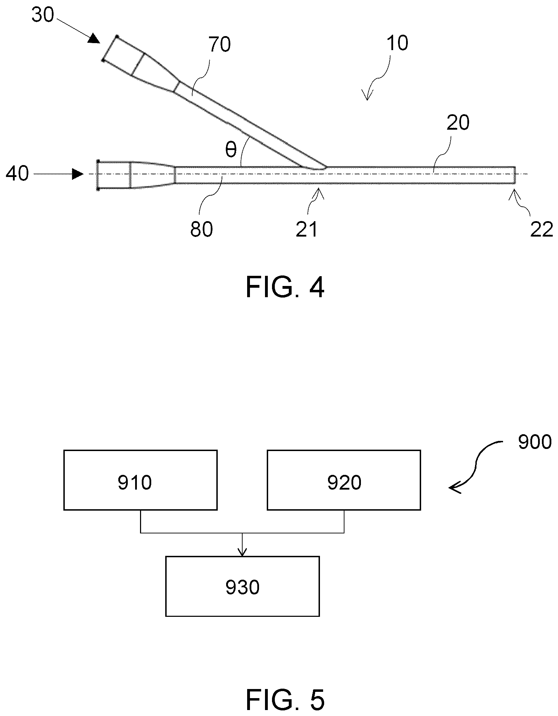

Figure 4 shows an example of the low-pressure nebulizer 10, which does not only comprise the tube 20, but also a gas inlet passage 70 and a liquid inlet passage 80. One (distal) end of the gas inlet passage 70 and one (distal) end of the liquid inlet passage 80 connect to the proximal inlet end 21 of the tube 20. The gas inlet passage 70 supplies the pressurized gas 30 to the tube 20 at the proximal inlet end 21, while the liquid inlet passage 80 supplies the liquid 40 to the tube 20 at the proximal inlet end 21. The gas inlet passage 70 connects to the gas lumen 50 at an inclination angle θ. In some examples, the angle θ is within a range of 15° to 65°.

The other (proximal) end of the gas inlet passage 70 may, for example, be connected to a gas tank or a regulated compressed gas source. If the gas inlet passage 70 is directly connected to a gas tank, a pressure regulator may be required to ensure the pressurized gas 30 is delivered at an appropriate and stable pressure.

The pressure of the pressurized gas 30 should be sufficiently high to effectively nebulize the liquid 40 when they exit and mix at the distal outlet end 22. However, as previously mentioned, excessively high gas pressure can lead to structural damage to delicate active agents in the liquid 40 during nebulization, potentially compromising their effectiveness.

To maintain precise control over the gas flow, a flow meter may be integrated to regulate and measure the volume of pressurized gas 30 entering the gas inlet passage 70. This allows for fine-tuning of gas delivery, optimizing the nebulization process while reducing potential adverse effects on the liquid formulation.

The type of pressurized gas 30 can vary depending on the liquid 40 being nebulized. For example, air is commonly used in general medical and home-use nebulizers because it is readily available and effective. Oxygen is often preferred for patients with respiratory conditions like COPD (chronic obstructive pulmonary disease) or severe asthma, where supplemental oxygen therapy is needed alongside medication delivery. In certain cases, heliox, i.e. a breathing gas mixture of helium and oxygen, may be used, especially for patients with severe airway obstruction, as its lower density helps improve airflow and allows the medication to reach deeper into the lungs.

The other (proximal) end of the liquid inlet passage 80 may, for example, be connected to a liquid chamber that accommodates the liquid 40 to be nebulized. To facilitate the movement of the liquid 40 from the liquid chamber into the liquid inlet passage 80, a compressed air source or another pressure-driven mechanism may be employed. Depending on the nebulizer design, additional components such as one-way valves, capillary tubes, or gravity-fed systems may also be incorporated to regulate liquid delivery and prevent backflow.

The composition of the liquid 40 can vary widely depending on the intended application of the nebulizer. For example, it may comprise liquid medications, such as mRNA vaccines, or biological formulations, such as protein-based therapeutics. Additionally, it could include cosmetic solutions, such as anti-aging serums, or agricultural products, such as plant growth hormones. The versatility of the nebulizer allows it to accommodate a broad range of liquid formulations across different industries, from pharmaceuticals and biotechnology to cosmetics and agriculture.

Different manufacturing methods can be employed. For example, the tube 20 can be integrally molded as a single piece using 3D printing technology or other suitable fabrication techniques.

Alternatively, the tube 20 may be produced in segments and then assembled into a single integrated piece. For example, one part of the tube 20, comprising the main proximal segment 61 of the liquid lumen 60, may be manufactured separately, while another part, comprising the contracted intermediate segment 62 and the expanded distal segment 63, is produced as a distinct piece. These parts can then be assembled to form the entire tube 20. This modular approach allows for greater flexibility in adjusting the pressure of the liquid 40. Instead of replacing the entire tube 20, only the part comprising the contracted intermediate segment 62 and the expanded distal segment 63, needs to be changed, while the rest of the tube remains reusable. This not only simplifies customization but also enhances cost efficiency and sustainability in production and usage.

Suitable materials for the tube 20 may include ultraviolet curable plastics, which offer excellent durability, chemical resistance, and precision molding capabilities. These materials are particularly well-suited for 3D printing, enabling the rapid and precise fabrication of complex geometries.

Depending on the specific application, other materials may also be considered to ensure compatibility with various nebulization environments and liquid formulations. These include medical-grade silicone, which provides flexibility and biocompatibility, biocompatible thermoplastics such as polycarbonate, polyethylene, or polypropylene, known for their strength and ease of processing, and fluoropolymers like Polytetrafluoroethylene (PTFE) and Fluorinated Ethylene Propylene (FEP), which offer superior chemical resistance and low friction properties.

Figure 5 is a block diagram of a method 900 to nebulize liquid formulations according to one example of the present disclosure.

In block 910, a pressurized gas 30 is conveyed in a gas lumen 50 from a proximal inlet end 21 to a distal outlet end 22 of a tube 20.

In block 920, a liquid 40 is conveyed in a liquid lumen 60 from the proximal inlet end 21 to the distal outlet end 22. The liquid 40 flows through a main proximal segment 61, a contracted intermediate segment 62 and an expanded distal segment 63, such that a velocity of the liquid 4 is increased in the contracted intermediate segment 62 relative to the main proximal segment 61, and the velocity of the liquid is decreased in the expanded distal segment 63 relative to the contracted intermediate segment 62.

In block 930, the pressurized gas 30 and the liquid 40 from the tube 20 are expelled at the distal outlet end 22, such that the expelled pressurized gas 30 and the expelled liquid 40 mix outside of the tube 20 to form an aerosol at the distal outlet end 22.

In some examples, the method 900 can be performed using a low-pressure nebulizer 10 according to any of the examples disclosed herein.

In some examples, in block 930 the pressurized gas 30 and the liquid 40 are expelled simultaneously and mix outside the tube 20 to form the aerosol at the distal outlet end 22.

In some examples, the liquid 40 is a liquid formulation. The liquid formulation comprises an active agent selected from the group consisting of: a nasal therapeutic agent, a pulmonary therapeutic agent, an ophthalmic therapeutic agent, a dermatological active ingredient, a biologic molecule, a gene therapy agent, a cell or tissue growth factor, an inhalable vaccine, a heat-sensitive biological agent, a sensitive flavoring agent, an essential oil, and a food-grade probiotic.

In some examples, a cleansing or rinsing nasal liquid formulation is nebulized to the nose, using examples of the disclosed low-pressure nebulizer 10 and/or following the disclosed method 900 to nebulize liquid formulations.

In some examples, a hydrating ophthalmic liquid formulation is nebulized to the eye, using the disclosed low-pressure nebulizer 10 and/or following the disclosed method 900 to nebulize liquid formulations.

In some examples, the disclosed low-pressure nebulizer 10 is used in food-grade applications, such as nebulizing a sensitive flavoring agent, an essential oil, and a probiotic, preferably following the disclosed method 900 to nebulize liquid formulations.

In some examples, the disclosed low-pressure nebulizer 10 is used in agricultural applications, such as nebulizing heat-sensitive biological agent over a crop, preferably following the disclosed method 900 to nebulize liquid formulations.

In some examples, the disclosed low-pressure nebulizer 10 comprises a liquid formulation comprising one of the following active agents:
a) a pulmonary therapeutic agent for use in the prevention and/or treatment of a pulmonary or systemic condition, wherein the prevention and/or treatment comprises administering the pulmonary therapeutic agent through a non-invasive pulmonary route using the nebulizer, or alternatively,
b) a nasal therapeutic agent, for use in the prevention and/or treatment of a nasal, neurological or systemic condition, wherein the prevention and/or treatment comprises administering the nasal therapeutic agent through a non-invasive nasal route using the nebulizer, or alternatively,
c) an ophthalmic therapeutic agent, for use in the prevention and/or treatment of an ophthalmic disease or condition, wherein the prevention and/or treatment comprises administering the ophthalmic therapeutic agent in the eyes using the nebulizer, or alternatively,
d) an inhalable vaccine for use in vaccination of a subject, wherein the use comprises administering the vaccine using the nebulizer, or alternatively,
e) a nucleic acid compound for use in gene therapy, or alternatively
f) a cell or tissue growth factor for use in tissue reparation, and
g) a dermatological therapeutic active ingredient, for use in the prevention and/or treatment of a skin disease or condition, wherein the prevention and/or treatment comprises administering the dermatological therapeutic agent in the skin using the nebulizer.

In these examples, the liquid formulations can be nebulized, for example, with the disclosed method 900 to nebulize liquid formulations.

Although only a number of examples have been disclosed herein, other alternatives, modifications, uses and/or equivalents of the present disclosure are possible. The scope of the present disclosure should not be limited by the particular examples but should be determined only by a fair reading of the claims that follow.

Reference signs related to drawings are placed in parentheses in the claims are solely for attempting to increase the intelligibility and shall not be construed as limiting the scope of the claim.

## Claims

1. A low-pressure nebulizer (10) for liquid formulations, comprising a tube (20), the tube (20) comprising:
∘ a proximal inlet end (21) configured for a pressurized gas (30) and a liquid (40) to enter the tube (20),
∘ a distal outlet end (22) configured for the pressurized gas (30) and the liquid (40) to exit the tube (20),
∘ a gas lumen (50) configured to convey the pressurized gas (30) from the proximal inlet end (21) to the distal outlet end (22),
∘ a liquid lumen (60) configured to convey the liquid (40) from the proximal inlet end (21) to the distal outlet end (22),
the low-pressure nebulizer (10) being configured such that the pressurized gas (30) and the liquid (40) are mixed outside the tube (20) at the distal outlet end (22) to form an aerosol,
wherein the liquid lumen (60) comprises:
- a main proximal segment (61) with a first length and a first cross section,
- a contracted intermediate segment (62) with a second length and a second cross section,
- an expanded distal segment (63) with a third length and a third cross section,
wherein the second cross section is smaller than the first cross section, and the third cross section is greater than the second cross section.

2. The low-pressure nebulizer (10) according to claim 1, wherein a longitudinal central axis of the gas lumen (50) and a longitudinal central axis of the liquid lumen (60) are parallel to a longitudinal central axis of the tube (20).

3. The low-pressure nebulizer (10) according to claim 2, wherein the tube (20) comprises two or more gas lumens (50), and the two or more gas lumens (50) are distributed around the liquid lumen (60).

4. The low-pressure nebulizer (10) according to claim 3, wherein the longitudinal central axis of the liquid lumen (60) coincides with the longitudinal axis of the tube (20), and the two or more gas lumens (50) are symmetrically distributed around the liquid lumen (60).

5. The low-pressure nebulizer (10) according to any one of the preceding claims, wherein the second cross section of the contracted intermediate segment (62) linearly decreases in distal direction within at least part of the second length with a constant decreasing slope between 5° and 50°.

6. The low-pressure nebulizer (10) according to any one of the preceding claims, wherein the third cross section of the expanded distal segment (63) linearly increases in distal direction within at least part of the third length with a constant increasing slope between 5° and 50°.

7. The low-pressure nebulizer (10) according to any one of the preceding claims, wherein a cross section of the liquid lumen (60) is circular.

8. The low-pressure nebulizer (10) according to any one of the preceding claims, wherein a cross section of the gas lumen (50) is circular or C-shaped.

9. The low-pressure nebulizer (10) according to any one of the preceding claims, wherein the second length of the contracted intermediate segment (62) is less than 10% of an entire length of the liquid lumen (60), and the third length of the expanded distal segment (63) is less than 15% of the entire length of the liquid lumen (60).

10. The low-pressure nebulizer (10) according to any one of the preceding claims, further comprising a pressurized gas inlet passage (70) configured to provide the pressurized gas (30) to the tube (20), wherein the gas inlet passage (70) connects to the gas lumen (50) at an inclination angle between 15° and 65°

11. The low-pressure nebulizer (10) according to any one of the preceding claims, wherein the tube (20) is manufactured as one integral piece.

12. A method to nebulize liquid formulations, comprising:
• conveying a pressurized gas (30) in a gas lumen (50) from a proximal inlet end (21) to a distal outlet end (22) of a tube (20),
• conveying a liquid (40) in a liquid lumen (60) from the proximal inlet end (21) to the distal outlet end (22) of the tube (20), wherein the liquid (4) flows through a main proximal segment (61), a contracted intermediate segment (62) and an expanded distal segment (63) of the liquid lumen (60), such that a velocity of the liquid (40) is increased in the contracted intermediate segment (62) relative to the main proximal segment (61), and the velocity of the liquid is decreased in the expanded distal segment (63) relative to the contracted intermediate segment (62),
• expelling, at the distal outlet end (22), the pressurized gas (30) and the liquid (40) from the tube (20), such that the expelled pressurized gas (30) and the expelled liquid (40) mix outside of the tube (20) to form an aerosol at the distal outlet end (22).

13. The method to nebulize liquid formulations according to claim 12, comprising using a low-pressure nebulizer (10) according to any one of claims 1-11.

14. The method to nebulize liquid formulations according to claim 12 or 13, wherein the liquid (40) is a liquid medication.

15. The method to nebulize liquid formulations according to any one of claims 12-14, wherein a pressure of the pressurized gas is below 1.0 bar.
